# EUROPÄISCHE PATENTSCHRIFT

(11) **EP 0 564 061 B1**
(45) Veröffentlichungstag und Bekanntmachung des Hinweises auf die Patenterteilung: **04.11.1998**
(21) Anmeldenummer: 93250092.9
(22) Anmeldetag: 30.03.1993
(51) Int. Cl.: C12M 3/06, C12M 3/04, C12P 21/08

(54) **Verfahren und Vorrichtung zur in vitro-Produktion von hochkonzentrierten monoklonalen Antikörpern**
Method and apparatus for the in-vitro production of monoclonal antibodies high concentration
Méthode et appareil pour la production in vitro d'anticorps monoclonaux à haute concentration

(30) Priorität: 31.03.1992 DE 4211169
(43) Veröffentlichungstag der Anmeldung: 06.10.1993
(73) Patentinhaber: GESKE & KRETZSCHMAR GBR, D-14163 Berlin (DE)
(72) Erfinder: Kretzschmar, Klaus-Günter, W-1000 Berlin 28 (DE); Geske, Tuula, W-1000 Berlin 37 (DE); L'age-Stehr, Johanna, W-1000 Berlin 37 (DE)

(56) Entgegenhaltungen:
- EP-A- 0 258 795
- WO-A-92/22634
- FR-A- 2 166 590
- GB-A- 2 002 814
- US-A- 5 026 650

## Beschreibung

Die Erfindung betrifft das im Anspruch 7 angegebene Verfahren zur in vitro-Produktion hochkonzentrierter monoklonaler Antikörper und eine Vorrichtung zur Durchführung dieses Verfahrens nach den Ansprüchen 1 bis 6, die durch eine erfindungsbedingte Taumelrotation die Langzeitkultivierung von Säugetierzellen in stabiler Suspension (in der Schwebe) erlaubt.

Monoklonale Antikörper sind heute in der medizinischen und biologischen Forschung nicht mehr wegzudenken. An universitären und anderen Forschungslaboratorien ist für die Produktion größerer Mengen solcher Antikörper immer noch die Ascites-Maus das am häufigsten verwendete Produktionsverfahren, obwohl dies auch in Deutschland tierschutzgesetzlich im Regelfall nicht mehr, oder nur in wenigen, einzeln genehmigungspflichtigen Ausnahmefällen zulässig ist.

Beim in vivo-Verfahren werden die Zellen, besonders die Asciteszellen oder Zellen im Gewebeverband, wesentlich effektiver mit wachstumsfördernden Nährstoffen versorgt und von schädlichen Stoffwechselprodukten entsorgt. Dadurch verläuft die Produktion von Antikörpern in der Ascites-Flüssigkeit optimal und die Antikörper-Konzentrationen steigen pro ml auf die ca. 100-1000 fach höheren Werte als in den üblichen Zellkulturüberständen an.

Vor einer diesbezüglichen Änderung der Tierschutzgesetze in den meisten Ländern wurden die Ascites-Mäuse zur Gewinnung möglichst großer Mengen von Antikörpern auch mehrmals punktiert. Nach den in den meisten europäischen Ländern sinngemäß angeglichenen Tierschutzrichtlinien vom "United Kingdom Coordinating Committee on Cancer Research", darf eine Maus jetzt nur noch einmal punktiert werden und das gesamte Ascitesvolumen darf maximal 20% des Körpergewichtes der Maus betragen. Es können auch bei der Erteilung von Ausnahmegenehmigungen also nur geringe Ascitesmengen pro Maus gewonnen werden.

Neben dem in vivo-Verfahren sind mehrere in vitro Herstellungsverfahren von monoklonalen Antikörpern beschrieben worden, wie stationäre Kultur in Flaschen, Spinner- und Rollerkulturen, Kapillar-und Perfusionsreaktoren, Hohlfaserperfusoren und Dialyseverfahren. Für den Laborbedarf sind die meisten dieser Verfahren nicht geeignet. Stationäre Kultur in Flaschen, Spinner- und Rollerkulturen lassen keine höheren Antikörperkonzentrationen als 0,01-0,1 mg/ml erreichen. Bei den anschließend nötigen aufwendigen Konzentrierungsverfahren kommt es zu einem relativ hohen Beimengung von Fremdproteinen aus dem Nährmedium. Kapillar- und Perfusionsreaktoren, Hohlfaserperfusoren und Dialyseverfahren erfordern in der Anschaffung und im Gebrauch teure komplizierte Geräte oder Einrichtungen. Diese benötigen darüberhinaus teure Perfusions- und Schlauchsysteme, die in der Anwendung leicht durch Pilze oder Bakterien kontaminiert werden können, nicht sterilisierbar und damit nicht wiederverwendbar sind. Solche aufwendigen Zellkulturanlagen, z.B. Hohlfaserperfusoren, sind meist nur für die industrielle Nutzung für eine Antikörpercharge pro Zeit geeignet. Sie Können in vitro Antikörperkonzentrationen erreichen, die im Bereich von Ascites (> 1 mg/ml) liegen.

Auch das Verfahren nach der Dialyseschlauch-Rollkultur-Methode ("Glasmaus") für den Laborbedarf erfüllt in der Praxis nicht die Forderung nach hohen Antikörperkonzentrationen. Die einfache Rotationsbewegung und den dadurch bedingte niedrige Mediumspiegel in der Glasmaus lassen die Zellen in den Dialyseschläuchen sedimentieren, Gradienten bilden oder verklumpen, was zu Zellwachstumstörungen und zu einer mangelnden Antikörperproduktion führt.

Der Erfindung liegt die Aufgabe zugrunde, ein Verfahren und eine Vorrichtung zu entwickeln, mit der eine einfach zu handhabende, preisgünstige in-vitro Herstellung von monoklonalen Antikörpern aus Hybridomazellen in höheren Konzentrationen ermöglicht wird und die eine echte Alternativmethode zur Ascitesmaus darstellt.

Erfindungsgemäß erfolgt das Verfahren unter Schütteln der Nährlösung und der Suspension durch Überlagerung mehrerer Bewegungsrichtungen.

Erfindungsgemäß besteht die Vorrichtung aus einer Taumelrotations-Dialysekammer und zeichnet sich durch einen Zylinder aus Polycarbonat aus, der mit zwei Deckscheiben aus dem gleichen Material abgeschlossen ist. Die Nut für die Aufnahme des Zylinders auf den Deckscheiben hat ihren Mittelpunkt neben dem Deckscheibenmittelpunkt und die Deckscheiben sind asymmetrisch um 180° zueinander versetzt befestigt. Die Deckscheiben können gleiche aber auch unterschiedliche Abmessungen im Durchmesser und in der Gestaltung des Umfangs aufweisen. So werden je nach gewünschter Bewegung der die Dialyseschläuche umspülenden Nährlösunge z.B. runde oder unrunde Deckscheiben eingesetzt. Die Vorrichtung stellt das Kulturgefäß dar.

Diese Anordnung der Deckscheiben bewirkt bei Bewegung des Kulturgefäßes auf der Rollerapparatur eine Schräglage und deshalb eine taumelnde Rotationsbewegung des Zylinders. Dadurch werden die Zellen in den Dialyseschläuchen in einer zellphysiologisch günstigen, stabilen Suspension gehalten und es kommt nicht zur Sedimentierung und Verklumpung.

Der Zylinder ist durch eine zentrale Mittel- und Spannachse und durch die in den Deckscheiben befindliche Nut mit Dichtung fixiert. Eine Deckscheibe weist zusätzlich mehrere Durchgänge auf. Zwei Durchgänge dienen der Aufnahme von Kanülen zur Entlüftung und für die Mediumzu- und abgabe bzw. Begasung. Weitere Durchgänge sind für die Anordnung der auf Spezialversorgungskanülen montierten Dialyseschläuche vorgesehen. In den Dialyseschläuchen wachsen die Zellen in stabiler Suspension und produzieren die monoklonalen Antikörper. Die Anordnung mehrerer Dialyseschläuche ermöglicht es einmal, in einer Kammer eine große Menge an einer Sorte an Antikörperkonzentraten zu gewinnen. Gleichzeitig besteht aber auch, z.B. bei Forschungsaufgaben, die möglichkeit, in jedem Dialyseschlauch gleichzeitig einen anderen Antikörper zu produzieren.

Durch die Kompartimentierung der Zellen in den Dialyseschläuchen werden die Zellen durch die Membran hindurch gut mit Nährstoffen aus dem Innenkammermedium versorgt, während gleichzeitig Abbau- und Stoffwechselprodukte dahin abgeführt werden.

Die neu konstruierten Spezialversorgungskanülen sind mit seitlichen Öffnungen im Schaft und mit Schließvorrichtungen versehen, die sowohl die Bedienung wie Füllung und Entnahme kontaminationssicher und einfach gestalten, als auch die Erzeugung eines diffusionsfördernden, leichten Überdrucks in den Dialyseschläuchen ermöglichen. An der anderen Seite der Schließvorrichtungen an den Versorgungskanülen sind Aufnahmestutzen mit Luer-Ansatz vorgesehen, an die man in der Medizin übliche sterile Einmalspritzen, Sterilfilter, Infusionsschlauchsysteme oder sterile Durchstichsepten zwecks gefahrloser Probenentnahme unter "nicht-sterilen" Bedingungen anschließen kann.

Erfindungswesentlich ist, daß alle Bauteile wiederverwendbar, leicht und sicher zu reinigen und autoklavierbar sind. Das als Material für die Vorrichtung verwendete glasklare Polycarbonat zeichnet sich durch seine große Robustheit gegenüber dem bruchempfindlichen und teuren Borosilikatglas aus und erlaubt ebenfalls eine optische Kontrolle während der Kultivierung.

Das Kulturgefäß besteht aus wenigen, einfach gestalteten und leicht ersetzbaren Einzelteilen. Die Handhabung benötigt keine Spezialausbildung und keine Spezialausrüstung des Labors.

Das Medium in der Innenkammer kann je nach Bedarf der Zellkultur durch die entsprechenden Kanülen in der Deckplatte mit einem CO₂/O₂ bzw. - Luft-Gemisch begast, ausgetauscht oder ergänzt werden und benötigt kein fötales Kälberserum (FKS). Der Mediumwechsel ist durch den Einsatz einer Pumpe leicht durchzuführen. Die Inkubation des Kulturgefässes erfolgt in einem Brutschrank oder Brutraum auf einer üblichen Rollerapparatur für Zellkulturflaschen.

Die Erfindung erlaubt die Langzeit-Kultivierung von Zellen in stabiler Suspension (in der Schwebe) und damit die Produktion von hohen Zelldichten und Konzentrationen von ins Medium abgegebenen Zellprodukten, z.B. von monoklonalen Antikörpern.

Durch die Erfindung wird gegenüber den üblichen in vitro-Zellkulturverfahren eine etwa 100-fache Anreicherung von gewünschten Zellprodukten ohne gleichzeitige Konzentration von unerwünschten Nährmediumbestandteilen erreicht.
Die Erfindung soll nachfolgend an einem Ausführungsbeispiel an Hand der Zeichnungen näher erläutert werden
Es zeigen:
- Fig.1:: Die Taumelrotations-Dialysekammer
- Fig.2:: Die obere Deckscheibe mit Bohrungen für die Versorgungsanschlüsse
Die Dialyseschläuche **2** werden zur Entfernung von eventuell vorhandenen Schwermetallkontaminationen vorbehandelt, alle Bauteile gewaschen und gut mit destilliertem Wasser gespült.

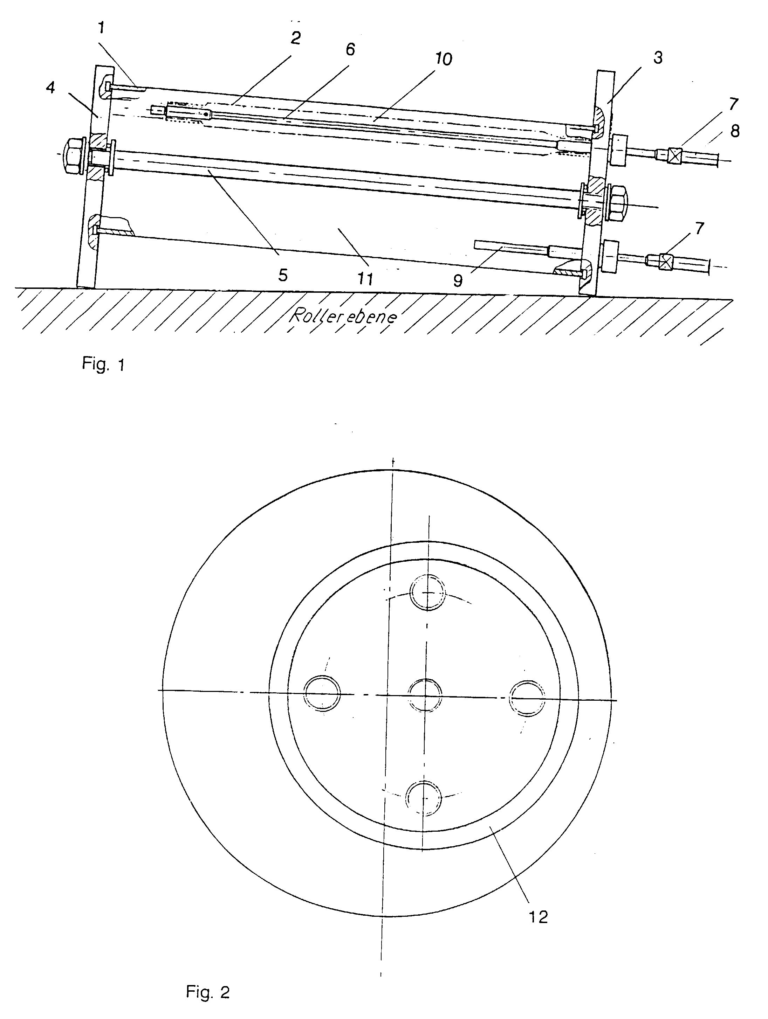

Aus dem Zylinder **1**, den beiden Deckscheiben **3**, **4** mit der Abdichtungsnut **12** und der Spannachse **5** wird die Taumelrotations-Dialysekammer zusammemgesetzt. Die Dialyseschläuche **2**, von denen einer in **Fig. 1** eingezeichnet ist, werden über die Spezialversorgungskanülen **6** gezogen und zur Bildung der Suspensions-Zellkammern **10** an den oberen und unteren Kanülenverdickungen mit Silikonschlauchstücken befestigt. Danach werden die unteren Reinigungsöffnungen der Spezialversorgungskanülen **6** mit Stopfen verschlossen und die so montierten, noch leeren Suspensionszellkammern **10** durch eine der Versorgungsbohrungen der oberen Deckscheibe **3** gesteckt, lose eingeschraubt und über die zentrale Spezialversorgungskanüle **6** mit destilliertem Wasser gefüllt. Die fertige Taumelrotations-Dialysekammer als Kulturgefäß wird autoklaviert und anschließend in eine sterile Werkbank gebracht. Mittels einer Pumpe wird die Innenkammer **11** mit Versorgungsmedium ohne Serum durch eine der Kanülen **9** gefüllt und auf Dichtigkeit geprüft. Nach dem Abziehen des Wassers aus den Dialyseschläuchen **2** wird die Zellsuspension, in Medium mit geringen Serumzusätzen, über die Spezialversorgungskanüle **6** mit einer sterilen Einmalspritze eingefüllt, wobei der Hahn **7** der Entlüftungskanüle **9** geöffnet ist. Nach Prallfüllung des Dialyseschlauches **2** wird bei leichtem Überdruck der Hahn **7** der Spezialversorgungskanüle **6** geschlossen. Nach Verschließen aller Hähne **7** wird das Kulturgefäß in erfindungsgemäßer Weise auf einer Rollerapparatur in einem Brutschrank bewegt. Je nach Bedarf der eingesetzten Zellsuspension wird die Innenkammer mit einem CO₂/O₂ bzw. - Luft-Gemisch begast sowie Mediumwechsel vorgenommen.

## Patentansprüche

1. Vorrichtung zur in vitro Produktion hochkonzentrierter monoklonaler Antikörper, **dadurch gekennzeichnet,** daß die als Kulturgefäß dienende Taumelrotations-Dialysekammer aus einem Zylinder (1) aus bruchsicherem Polycarbonat besteht, der an den Enden durch mit einer Nut (12) versehenen Deckscheiben (3, 4) verschlossen ist, die Deckscheiben (3, 4) asymmetrisch um 180° zueinander versetzt angeordnet sind, der Mittelpunkt der Nut (12) neben dem Mittelpunkt der Deckscheiben (3, 4) liegt, die Deckscheibe (3) mehrere Öffnungen für das Anordnen von Versorgungsleitungen (6, 9) besitzt und die Versorgungsleitungen (6) von Dialyseschläuchen (2) umgeben sind.

2. Vorrichtung nach Anspruch 1, **dadurch gekennzeichnet,** daß die Deckscheiben (3, 4) gleiche Abmessungen aufweisen.

3. Vorrichtung nach Anspruch 1, **dadurch gekennzeichnet**, daß die Deckscheiben (3, 4) unterschiedliche Abmessungen aufweisen.

4. Vorrichtung nach Anspruch 1 bis 3, **dadurch gekennzeichnet,** daß die Deckscheiben (3, 4) rund sind.

5. Vorrichtung nach Anspruch 1 bis 3, **dadurch gekennzeichnet,** daß die Deckscheiben (3, 4) unrund sind.

6. Vorrichtung nach Anspruch 1 bis 5, **dadurch gekennzeichnet,** daß die Versorgungsleitungen (6, 9) außerhalb der Taumelrotations- Dialysekammer mit Schließvorrichtungen (7) versehen sind.

7. Verfahren zur in vitro Produktion hochkonzentrierter monoklonaler Antikörper unter Verwendung der Vorrichtung gemäß Anspruch 1.

## Revendications

1. Le dispositif pour la production des anticorps monoclonaux *in vitro* en concentration forte **est charactérisé** du fait que la chambrede dialyse à rotation excentrique qui sert comme vase de culture est composé d'un cylindre (1) de polycarbonate incassable qui est clôturé aux bouts avec des couvercles protectifs (3, 4) avec une rainure (12); les couvercles protectifs sont fixés asymmetriquement et contrariés de 180°. Le centre de la rainure (12) est à coté du centre des couvercles protectifs (3,4), les couvercles protectifs ont quelques orifices pour l'arrangement des tuyaux d'alimentation (6,9) et les tuyaux d'alimentation (6) sont entourrés par des tuyaux de dialyse (2).

2. Le dispositif après titre 1 **est characrerisé** par le fait que les couvercles protectifs (3,4) ont les mêmes métrages.

3. Le dispositif après titre 1 **est characrerisé** par le fait que les couvercles protectifs (3,4) ont des métrages différents.

4. Le dispositif après les titres 1-3 **est characrerisé** par le fait que les couvercles protectifs (3,4) sont ronds.

5. Le dispositif après les titres 1-3 **est characrerisé** par le fait que les couvercles protectifs (3,4) sont non-ronds

6. Le dispositif après les titres 1-5 **est characrerisé** par le fait que les connexions de tuyaux (6,9) sont prévues avec un mécanisme de clôture en déhors de la chambre-de dialyse à rotation excentrique.

7. Le procédé pour la production *in vitro* des anticorps monoclonaux à concentration forte sous utilisation du dispositif selon titre 1.

## Claims

1. The device for *in vitro* production of high concentrated monoclonal antibodies is **characterised** by the fact that the tumbling rotation dialysis chamber which serves as culture vessel consists of a cylinder (1) of unbreakable polycarbonate closed at the ends by cover plates (3, 4) with circular slots (12), the cover plates (3, 4) are fixed asymmetrically by 180° to each other, the focus of the slot is next to the focus of the cover plates (3, 4) the cover plate (3) has several openings for the arrangement of supply tubing (6, 9) and the supply tubings (6) are surrounded by dialysis tubes(2).

2. The device according to claim 1 is **characterised** by the fact that the cover plates (3, 4) have the same measurements.

3. The device according to claim 1 is **characterised** by the fact that the cover plates (3, 4) have different measurements.

4. The device according to claim 1 to 3 is **characterised** by the fact that the cover plates (3, 4) are round.

5. The device according to claim 1 to 3 is **characterised** by the fact that the cover plates (3, 4) are non-rounded.

6. The device according to claim 1 to 5 is **characterised** that the supply tubing (6, 9) outside the tumbling rotation dialysis chamber are equipped with closing mechanisms (7).

7. Procedures for *in vitro* production of highly concentrated monoclonal antibodies by utilisation of the device according to claim 1.
